# EUROPÄISCHE PATENTSCHRIFT

(11) **EP 3 725 366 B1**
(45) Veröffentlichungstag und Bekanntmachung des Hinweises auf die Patenterteilung: **15.12.2021**
(21) Anmeldenummer: 19180525.8
(22) Anmeldetag: 17.06.2019
(51) Int. Cl.: A61N 1/375, H01R 4/50, H01R 13/24, H01R 24/58, H01R 39/64

(54) **ELEKTRISCHES KONTAKTBAUTEIL**
ELECTRICAL CONTACT COMPONENT
COMPOSANT DE CONTACT ÉLECTRIQUE

(30) Priorität: 17.04.2019 EP 19169882
(43) Veröffentlichungstag der Anmeldung: 21.10.2020
(73) Patentinhaber: BIOTRONIK SE & Co. KG, 12359 Berlin (DE)
(72) Erfinder: Hartmann-Bax, Kathy, 14947 Nuthe-Urstromtal (DE); Wali, Ruien, 10559 Berlin (DE)
(74) Vertreter: Biotronik Corporate Services SE

(56) Entgegenhaltungen:
- DE-A1-102016 217 673
- US-A1- 2013 197 603
- US-A1- 2016 164 195
- US-B2- 6 498 952

## Beschreibung

Die vorliegende Erfindung betrifft ein elektrisches Kontaktbauteil für ein medizinisches Implantat.

Medizinische Implantate, insbesondere aktive medizinische Implantate, weisen in der Regel therapeutische und/oder diagnostische Elektroden auf, die bestimmungsgemäß im Zielgewebe elektrische Impulse aufnehmen oder abgeben. Zur Gewährleistung der Funktion des Implantats ist es erforderlich, die Elektrode sicher und zuverlässig mit den Komponenten im Inneren des Implantats, insbesondere mit der Energiequelle und den Steuerschaltkreise, elektrisch zu kontaktieren.

Hinsichtlich der Kontaktierung der Elektroden existieren internationale Standards, beispielsweise DF-4/IS-4 für 4-polige Steckerverbindungen bzw. Konnektoren. Eine Variante, um den IS4 Standard in aktiven Implantaten (IPG/ICD) zu realisieren, stellt ein elektrisches Kontaktbauteil der, welches aus einer rotationssymmetrischen Federhülse und einem in die Federhülse eingeschweißten Federelement besteht. Ein bevorzugtes Federelement ist beispielsweise eine schräggewickelte Spiralfeder, die zu einen Ringfeder bzw. Torusfeder verschweißt ist.

Die oben beschriebene Variante ist jedoch in der Herstellung und Montage aufwendig, insbesondere die Fertigung der Ring- bzw. Torusfeder, und schwer automatisierbar.

Es ist daher einer der Erfindung zu Grunde liegende Aufgabe, ein elektrisches Kontaktbauteil für die Steckeraufnahme bzw. Kontaktbuchse eines medizinischen Implantats zur Verfügung zu stellen, welches eine robuste und zuverlässige elektrische Kontaktierung ermöglicht und vergleichsweise einfach herstellbar ist.

Diese Aufgabe wird durch ein elektrisches Kontaktbauteil mit den Merkmalen des Anspruchs 1, eine Kontaktbuchse mit den Merkmalen des Anspruchs 13, und ein implantierbares Medizingerät mit den Merkmalen des Anspruchs 14 gelöst. Bevorzugte Ausführungsformen sind in den entsprechenden abhängigen Ansprüche und der folgenden Beschreibung angegeben.

Gemäß Anspruch 1 wird ein elektrisches Kontaktbauteil für eine Kontaktbuchse eines implantieren Medizingeräts zur Verfügung gestellt. Das elektrische Kontaktbauteil weist auf:
- ein elektrisches Kontaktelement zur elektrischen Kontaktierung eines elektrischen Gegenkontakts, und
- einen elektrisch leitfähigen Außenring mit einer Steckerbohrung zur Aufnahme des elektrischen Gegenkontakts, wobei der Außenring auf der Innenseite eine Aufnahmeeinrichtung zur Aufnahme des elektrischen Kontaktelements, insbesondere eine Nut, aufweist.

Erfindungsgemäß ist dabei vorgesehen, dass das elektrische Kontaktelement durch eine Vielzahl von elektrisch leitfähigen Kontaktkörpern und mindestens ein Käfigelement zur Aufnahme der elektrisch leitfähigen Kontaktkörper gebildet wird.

Bei dem elektrischen Gegenkontakt handelt es sich insbesondere um den Stecker einer Elektrode, die mit dem implantierbaren Medizingerät elektrisch verbunden werden soll.

Die Aufnahmeeinrichtung zur Aufnahme des elektrischen Kontaktelements ist insbesondere zur Positionierung und Führung des elektrischen Kontaktelements (Käfigelement und elektrisch leitfähige Kontaktkörper) ausgebildet.

Der Begriff Kontaktkörper im Sinne der vorliegenden Erfindung bezeichnet insbesondere jeden Körper, der dazu ausgebildet ist, den elektrischen Gegenkontakt elektrisch kontaktieren zu können.

Der Begriff Käfigelement im Sinne der vorliegenden Erfindung bezeichnet insbesondere eine Aufnahmeeinrichtung zur Aufnahme der elektrisch leitfähigen Kontaktkörper, welches dazu ausgebildet ist, die Kontaktkörper voneinander auf Abstand und in Position zu halten. Ein solches Käfigelement kann mehrere Aufnahmen für die elektrisch leitfähigen Kontaktköper aufweisen, wobei die elektrisch leitfähigen Kontaktkörper insbesondere das Käfigelement durchgreifen bzw. sich durch das Käfigelement oder den Käfig erstrecken, so dass die elektrisch leitfähigen Kontaktkörper den elektrischen Gegenkontakt elektrisch kontaktieren können. Hierbei können die Aufnahmen einen geringeren Durchmesser aufweisen, als der Durchmesser der darin angeordneten Kontaktkörper. Somit kann insbesondere gewährleistet werden, dass die Kontaktkörper innerhalb der Aufnahmeeinrichtung zur Aufnahme des elektrischen Kontaktelements, insbesondere innerhalb der Nut, durch das Käfigelement angeordnet bzw. fixiert werden. Vorzugsweise ist das Käfigelement bzw. dessen Aufnahmen derart ausgebildet, dass die Kontaktkörper einen vordefinierten Freiheitsgrad der Bewegung genießen, d. h. insbesondere, dass die Kontaktkörper sich innerhalb der Aufnahme bewegen können.

Gemäß einer Ausführungsform des erfindungsgemäßen elektrischen Kontaktbauteils ist vorgesehen, dass das elektrische Kontaktbauteil weiterhin ein Federelement aufweist, welches innerhalb der Aufnahmeeinrichtung zur Aufnahme des elektrischen Kontaktelements, insbesondere der Nut, zwischen den Kontaktkörpern und der Innenseite des Außenrings angeordnet ist, wobei das Federelement insbesondere dazu ausgebildet ist, immer mindestens einen der Kontaktkörper mit dem Außenring und dem elektrischen Gegenkontakt in einem elektrischen Kontakt zu halten, so dass ein ständiger elektrischer Kontakt zwischen dem elektrischen Gegenkontakt und dem Außenring besteht bzw. gewährleistet wird.

Alternativ kann das Federelement dazu ausgebildet sein, einen der Kontaktkörper und den Außenring und mindestens einen der Kontaktkörper und den elektrischen Gegenkontakt in elektrischen Kontakt zu halten, um so einen ständigen elektrischen Kontakt zwischen dem elektrischen Gegenkontakt und dem Außenring zu gewährleisten, sofern das Käfigelement selbst elektrisch leitfähig ist.

Hierbei kann das Federelement in beliebiger Lage oder Orientierung innerhalb der Aufnahmeeinrichtung zur Aufnahme des elektrischen Kontaktelements angeordnet sein, sofern mindestens einer der Kontaktkörper und der Außenring und mindestens einer der Kontaktkörper und der elektrischen Gegenkontakt in ständigen elektrischen Kontakt gehalten werden.

Insbesondere übt das Federelement auf den oder die Kontaktkörper eine Kraft aus, die in Richtung des elektrischen Gegenkontakts gerichtet ist.

Gemäß einer weiteren Ausführungsform des erfindungsgemäßen elektrischen Kontaktbauteils ist vorgesehen, dass der elektrisch leitfähige Außenring und das mindestens eine Käfigelement stoffschlüssig gefügt sind, insbesondere durch Verpressen, Verkleben, Verlöten, Vernieten, Punkten oder Verschweißen.

Gemäß einer Ausführungsform des erfindungsgemäßen elektrischen Kontaktbauteils ist vorgesehen, dass der elektrisch leitfähige Außenring und das mindestens eine Käfigelement aus einem Metall oder einer Metalllegierung bestehen. Vorteilhafterweise können damit Außenring und Käfigelement einfacher gefügt werden, insbesondere durch Schweißen oder Löten. Gleichzeitig kann dabei auch das Käfigelement zur Herstellung eines zuverlässigen elektrischen Kontakts zwischen Außenring und elektrischen Gegenkontakt beitragen.

Gemäß einer Ausführungsform des erfindungsgemäßen elektrischen Kontaktbauteils ist vorgesehen, dass das Käfigelement innerhalb der Aufnahmeeinrichtung, insbesondere innerhalb der Nut angeordnet ist.

Gemäß einer weiteren Ausführungsform, weist das erfindungsgemäßen elektrische Kontaktbauteil nur ein Käfigelement auf.

Gemäß einer Ausführungsform des erfindungsgemäßen elektrischen Kontaktbauteils ist vorgesehen, dass der Außenring ein Gehäuse und einen Deckel aufweist, wobei das Gehäuse und der Deckel jeweils einen Steg auf der Innenseite aufweisen, und die Stege mit der Innenseite des Gehäuses die Aufnahmeeinrichtung zur Aufnahme des elektrischen Kontaktelements, insbesondere die Nut, bilden.

Gemäß einer weiteren Ausführungsform des erfindungsgemäßen elektrischen Kontaktbauteils ist vorgesehen, dass das Gehäuse mit dem Deckel stoffschlüssig gefügt ist, insbesondere durch Verpressen, Verkleben, Verlöten, Vernieten, Punkten oder Verschweißen.

Gemäß einer weiteren Ausführungsform des erfindungsgemäßen elektrischen Kontaktbauteils ist vorgesehen, dass die Aufnahmeeinrichtung zur Aufnahme des elektrisch leitfähigen Kontaktelements, insbesondere die Nut, eines der folgenden Formen aufweist: prismaförmig, rechteckig, trapezförmig, halbrund, viertelrund oder dreieckig.

Gemäß einer weiteren Ausführungsform des erfindungsgemäßen elektrischen Kontaktbauteils ist vorgesehen, dass die elektrisch leitfähigen Kontaktkörper als Kugeln oder Rollen ausgebildet sind, wobei insbesondere das Käfigelement entsprechend als Rollenkäfig oder Kugelkäfig ausgebildet ist.

Gemäß einer weiteren Ausführungsform des erfindungsgemäßen elektrischen Kontaktbauteils ist vorgesehen, dass das Federelement als ringförmiges Federelement ausgebildet ist. Vorteilhafterweise kann das Federelement als ein geschlossenes ringförmiges Federelement ausgebildet sein.

Gemäß einer weiteren Ausführungsform des erfindungsgemäßen elektrischen Kontaktbauteils ist vorgesehen, dass das ringförmige Federelement einen der folgenden Querschnitte aufweist: rund (ringförmiges Rohr), halbrund, s-förmig, z-förmig, c-förmig, rechteckig oder omega-förmig.

Gemäß einer weiteren Aufführungsform des erfindungsgemäßen elektrischen Kontaktbauteils ist vorgesehen, dass an jedem Kontaktkörper des elektrischen Kontaktelements ein Federelement angeordnet ist, wobei das jeweilige Federelement zwischen den jeweiligen Kontaktkörper und der Innenseite des Außenrings angeordnet ist.

Gemäß einer weiteren Ausführungsform des erfindungsgemäßen Kontaktbauteils ist vorgesehen, dass der jeweilige Kontaktkörper mit dem jeweiligen Federelement stoffschlüssig gefügt oder einstückig ausgebildet ist.

Gemäß einer weiteren Ausführungsform des erfindungsgemäßen elektrischen Kontaktbauteils ist vorgesehen, dass das an dem Kontaktkörper angeordnete Federelement als federndes Druckstück ausgebildet ist.

Insbesondere wird hierbei das federnde Druckstück durch das Federelement und eine Hülse ausgebildet, wobei das Federelement innerhalb der Hülse angeordnet ist. Vorzugsweise ist auch der an dem Federelement angeordnete Kontaktkörper in der Hülse angeordnet, wobei insbesondere der Kontaktkörper und das federnde Druckstück zusammen als Federkugel ausgebildet sind. Die oben beschriebene Hülse kann hierbei auch als ein individuelles Käfigelement dienen, wodurch auf ein Käfigelement für alle elektrisch leitfähigen Kontaktkörper verzichtet werden kann.

Gemäß einer weiteren Ausführungsform des erfindungsgemäßen elektrischen Kontaktbauteils ist vorgesehen, dass das ringförmige Federelement als Spiralfeder ausgebildet ist.

Gemäß einer alternativen Ausführungsform des erfindungsgemäßen elektrischen Kontaktbauteils ist vorgesehen, dass das Käfigelement federelastisch ausgebildet ist. Vorteilhafterweise kann in dieser Ausführungsform auf ein zusätzliches Federelement verzichtet werden, da ein solcherart ausgestaltetes Käfigelement die Kontaktkörper in elektrischen Kontakt mit der Innenseite des Außenrings und dem elektrischen Gegenkontakt halten kann. Vorzugsweise ist dabei das Käfigelement als Blechkäfig ausgebildet und aus einem elektrisch leitfähigen Material geformt.

Gemäß einer weiteren Ausführungsform des erfindungsgemäßen elektrischen Kontaktbauteils ist vorgesehen, dass das Käfigelement als Blechkäfig oder Massivkäfig ausgebildet ist, wobei der Massivkäfig insbesondere als ringförmiges Band ausgebildet ist, welches eine Vielzahl von Öffnungen zur Aufnahme der Kontaktkörper aufweist, und der Durchmesser der Vielzahl von Öffnungen geringer ist, als der Durchmesser der Kontaktkörper.

Gemäß einer weiteren Ausführungsform des erfindungsgemäßen elektrischen Kontaktbauteils ist vorgesehen, dass das Käfigelement aus einem der folgenden Stoffe besteht oder umfasst: Kunststoff, insbesondere ein Duroplast, ein Thermoplast, ein Elastomer oder eine Kombinationen davon, Edelstahl, insbesondere Chrom-Nickel-Molybdän-Stahl, wie etwa 1.4435, eine Platiniridiumlegierung, eine Nickel-Kobalt-Legierung, insbesondere MP35N, Titan oder eine Titanlegierung, insbesondere Nitinol.

Gemäß einer weiteren Ausführungsform des erfindungsgemäßen elektrischen Kontaktbauteils ist vorgesehen, dass der Außenring Edelstahl, insbesondere Chrom-Nickel-Molybdän-Stahl, wie etwa 1.4435, eine Platiniridiumlegierung, eine Nickel-Kobalt-Legierung, insbesondere MP35N, Titan oder eine Titanlegierung umfasst oder daraus besteht.

Gemäß einer Ausführungsform des erfindungsgemäßen elektrischen Kontaktbauteils ist vorgesehen, dass die Kontaktkörper als Material Edelstahl, insbesondere Chrom-Nickel-Molybdän-Stahl wie etwa 1.4435, eine Nickel-Kobalt-Legierung, insbesondere MP35N, Titan oder eine Titanlegierung umfassen oder daraus bestehen.

Gemäß einer weiteren Ausführungsform des erfindungsgemäßen elektrischen Kontaktbauteils ist vorgesehen, dass das elektrische Kontaktelement 7 bis 15 Kontaktkörper aufweist.

Gemäß Anspruch 13, wird eine Kontaktbuchse für ein implantierbares Medizingerät zur Verfügung gestellt, welches mindestens ein elektrisches Kontaktbauteil gemäß Anspruch 1 oder einer der dazugehörigen Ausführungsformen aufweist.

Insbesondere weist die erfindungsgemäße Kontaktbuchse eine Steckeraufnahme zur Aufnahme eines elektrischen Gegenkontakts auf, insbesondere der Stecker einer Elektrodenleitung, wobei die Steckeraufnahme unter anderem durch das mindestens eine elektrische Kontaktbauteil gebildet wird. Die Kontaktbuchse bzw. das elektrische Kontaktbauteil dient dabei der Übertragung von elektrischen Signalen auf und von dem elektrischen Gegenkontakt bzw. dem Stecker der Elektrodenleitung.

Typischerweise ist eine solche Kontaktbuchse Bestandteil eines Headers eines implantierbaren Medizingeräts, wobei die Kontaktbuchse, insbesondere das erfindungsgemäße Kontaktbauteil, in elektrischem Kontakt mit den Komponenten des implantierbaren Medizingeräts steht, welche im Inneren des Gehäuse des implantierbaren Medizingeräts angeordnet sind.

Insbesondere ist dann das elektrische Kontaktbauteil in der erfindungsgemäßen Kontaktbuchse über einen elektrischen Leiter mit einer Durchführung des implantierbaren Medizingeräts elektrisch in Kontakt. Bei dem elektrischen Leiter handelt es sich insbesondere um ein Leitungsband.

Alternativ dazu kann die Kontaktbuchse auch im Gehäuse eines implantierbaren Medizingeräts angeordnet sein, wobei in diesem Fall das betreffende Medizingerät vorzugsweise keinen Header aufweist. Vorteilhafterweise kann dabei das erfindungsgemäße elektrische Kontaktbauteil ohne hermetische Durchführung mit den Komponenten des implantierbaren Medizingeräts elektrisch kontaktiert werden.

Gemäß einer Ausführungsform der erfindungsgemäßen Kontaktbuchse ist vorgesehen, dass die Kontaktbuchse 1 bis 10, insbesondere 4 bis 8, der erfindungsgemäßen elektrischen Kontaktbauteile aufweist.

Gemäß Anspruch 14 wird ein implantierbares Medizingerät zur Verfügung gestellt, welches mindestens ein erfindungsgemäßes elektrisches Kontaktbauteil oder eine erfindungsgemäße Kontaktbuchse aufweist.

Typischerweise umfasst ein solches implantierbares Medizingerät ein hermetisches verschlossenes Gehäuse mit einer elektrischen Durchführung, wobei innerhalb des Gehäuses notwendige Komponenten wie Energiequelle, Steuerelektronik, Diagnoseelektronik und/oder Kondensator angeordnet sind, und die erfindungsgemäße Kontaktbuchse bzw. das erfindungsgemäße elektrische Kontaktbauteil im Header des implantierbaren Medizingeräts. Über die Durchführung werden dabei elektrische Signale zwischen den vorgenannten Komponenten und der Kontaktbuchse, oder genauer dem elektrischen Kontaktbauteil, ausgetauscht. Insbesondere ist die Kontaktbuchse bzw. das elektrische Kontaktbauteil über einen elektrischen Leiter, wie ein Leitungsband, mit der elektrischen Durchführung elektrisch verbunden.

Alternativ dazu kann die erfindungsgemäße Kontaktbuchse bzw. das erfindungsgemäße Kontaktbauteil im Gehäuse des implantierbaren Medizingerät, wobei das Medizingerät in diesem Fall keinen Header aufweist.

Gemäß einer Ausführungsform des erfindungsgemäßen implantierbaren Medizingeräts ist vorgesehen, dass das implantierbare Medizingerät als Herzschrittmacher, Kardioverter-Defibrillator oder Neurostimulator ausgebildet ist.

Im Folgenden werden Ausführungsbeispiele der Erfindung anhand von Figuren beschrieben. Diese dienen der Erläuterung der Erfindung, schränken jedoch nicht den Schutzumfang ein.

### Dabei zeigen

- Fig. 1: ein erfindungsgemäßes elektrisches Kontaktbauteil;
- Fig. 2: eine Detailansicht einiger Komponenten des erfindungsgemäßen elektrischen Kontaktbauteils;
- Fig. 3: einige Ausführungsformen des erfindungsgemäßen elektrischen Kontaktbauteils; und
- Fig. 4: alternative Ausführungsformen von Komponenten des erfindungsgemäßen elektrischen Kontaktbauteils.

Zur Kontaktierung einer IS4 oder DF4 Elektrode wird im Header eines IPGs oder ICDs ein elektrischer Gegenkontakt benötigt. Erfindungsgemäß ist insbesondere vorgesehen, statt der herkömmlichen Federkontakte ein Wälzlager 140, insbesondere Kugellager, als elektrischen Kontakt zu verwenden.

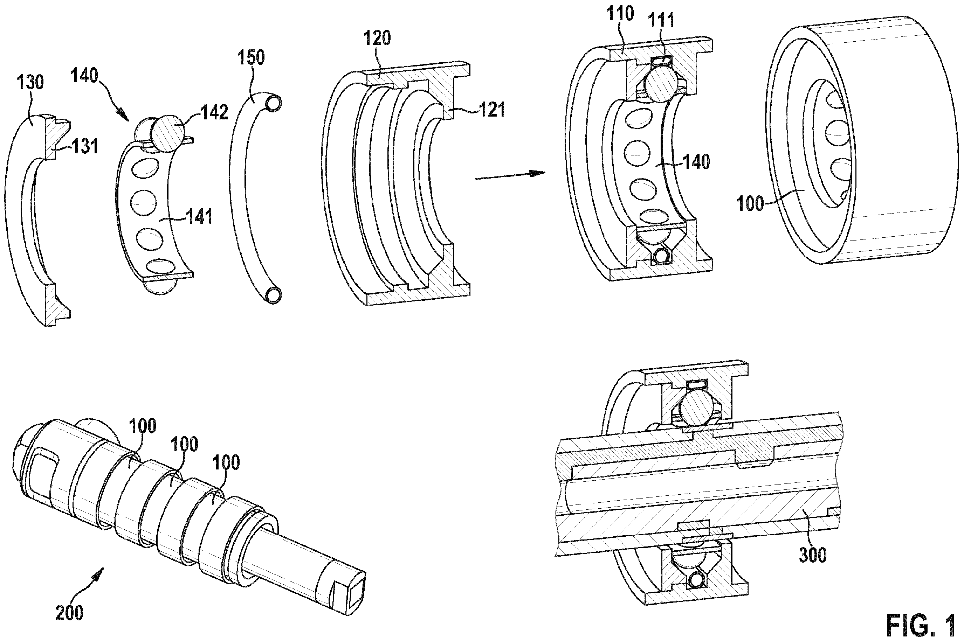

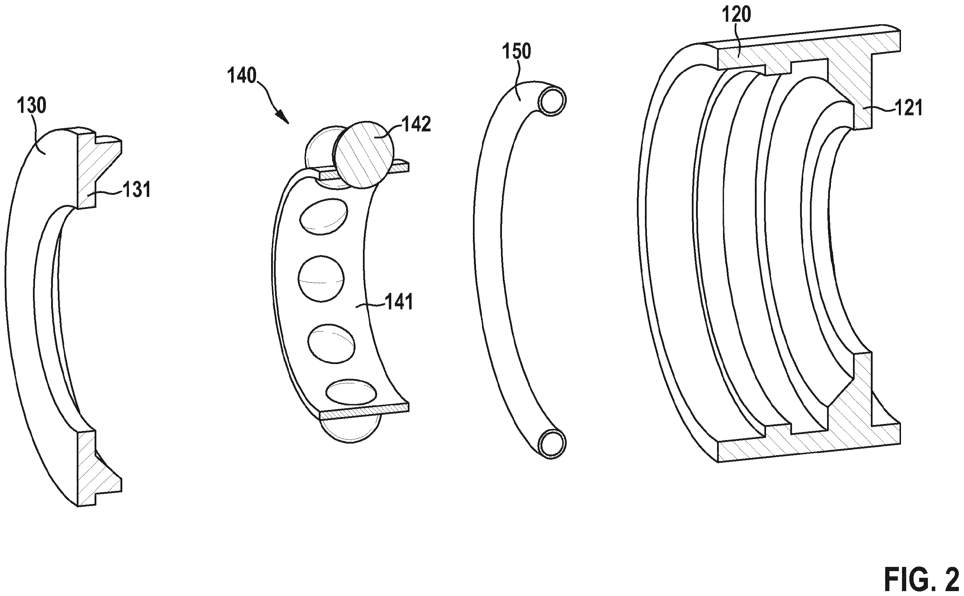

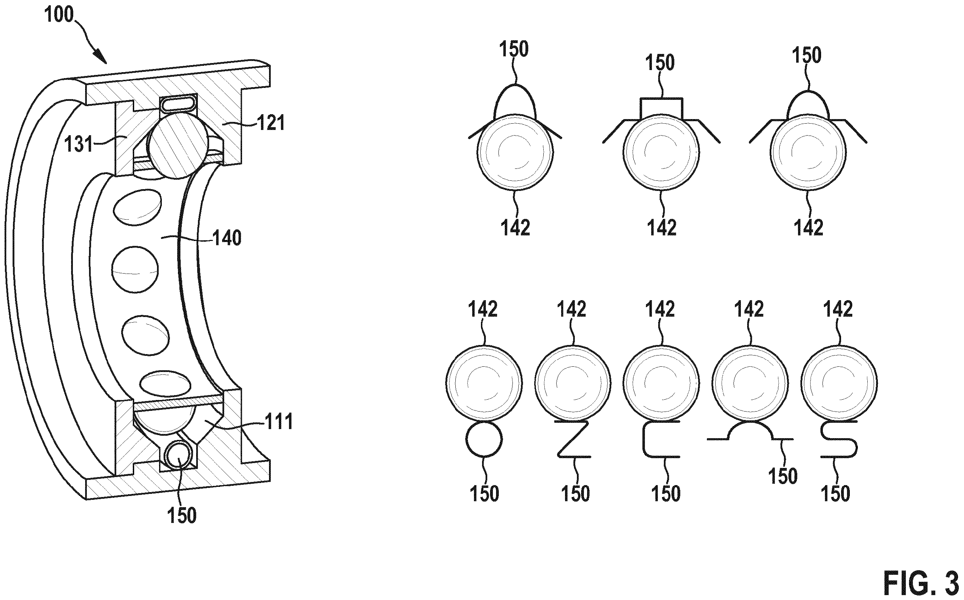

Wie in den Figuren 1 bis 3 dargestellt, umfasst demnach ein bevorzugtes erfindungsgemäßes elektrisches Kontaktbauteil 100 folgende Komponenten: einen äußeren Profilring 110, einen Federring 150, sieben bis fünfzehn Kugel 142, sowie einen mehrteiligen Käfig 141 zur Aufnahme der Kugeln 142.

Der äußere Profilring 110 ist so gestaltet, dass die Kugeln 142 mindestens einen elektrischen punktuellen Kontakt (vorzugsweise mehrere punktuelle Kontakte) zum Profilring 110 aufweisen. Die Innenkontur 111 des Profilringes 110 kann unterschiedliche Formen (Prisma, rechteckig, trapezförmig, rund, halbrund etc.). An der Außenseite des Profilrings 110 kann ein Verdrahtungsband (Zuführung der Energie / Strom) angeschweißt werden. Der Profilring 110 ist vorzugsweise ein klassisches Drehteil, kann aber auch durch MIM (metal injection molding) oder andere Formgebungsverfahren wie Drucken oder Verpressen und Sintern hergestellt werden. Der Profilring 110 kann einteilig aber auch zweiteilig 120, 130 ausgebildet sein. Soweit ein zweiteiliger Profilring 110 verwendet wird, sind beide Teile 120, 130 nach Montage der Kugeln 142 und des Käfigs 141 miteinander stoffschlüssig gefügt, beispielsweise durch Verpressen, Schweißen, Kleben, Löten, Nieten oder Punkten.

Der Federring 150 besteht aus einer einteiligen federnden Komponente, die zum einen den ständigen Kontakt zwischen Kugel 142 und Profilring 110 sicherstellt, zum anderen die Kugeln 142 in radialer Richtung (nach innen und außen) federn lässt. Der Federring 150 kann offen sowie geschlossen gestaltet sein. Querschnittsformen des Federrings 150 können folgenden Geometrien annehmen: o-/c-/s-/z-Form. Der Federring 150 kann auch eine geschlossene Spiralfeder sein.

Die Kugeln 142 sind dabei so dimensioniert, dass mit dem Stecken der Elektrode 300 ein elektrischer Kontakt zwischen Elektrode 300 und Profilring 110 (über dem Federring 150) realisiert wird.

Um die Kugeln 142 im Profilring 110 zu halten, wird ein Käfig 141 genutzt. Der Käfig 141 dabei hat die Aufgabe, die Kugeln 142 voneinander auf Abstand und Position zu halten. Der Käfig 141 kann in mindestens in radialer Richtung federelastisch sein für den Fall, dass der Federring 150 als Komponente weggelassen werden kann. Der Käfig 141 kann als geschlossener bzw. als geöffneter Ring realisiert sein und aus einem oder mehreren Teilen bestehen. Geeignete Materialien den Käfig sind beispielsweise Kunststoff (z.B. Polyamid), Nitinol, 1.4435, Platiniridium, MP35N, Titan, Edelstahl sowie alle weiteren Materialarten, welche die Anforderungen an ein elektrischen Kontakt im Dauerimplantat erfüllen.

Vorzugsweise ist der Außenring aus einer Platiniridiumlegierung und die Kugeln des Kugellagers aus MP35N, 1.4435 (Edelstahl) oder Titan.

Prinzipiell sind jedoch alle Materialpaarungen denkbar, welche die Anforderungen an einen elektrischen Kontakt im Dauerimplantat erfüllen.

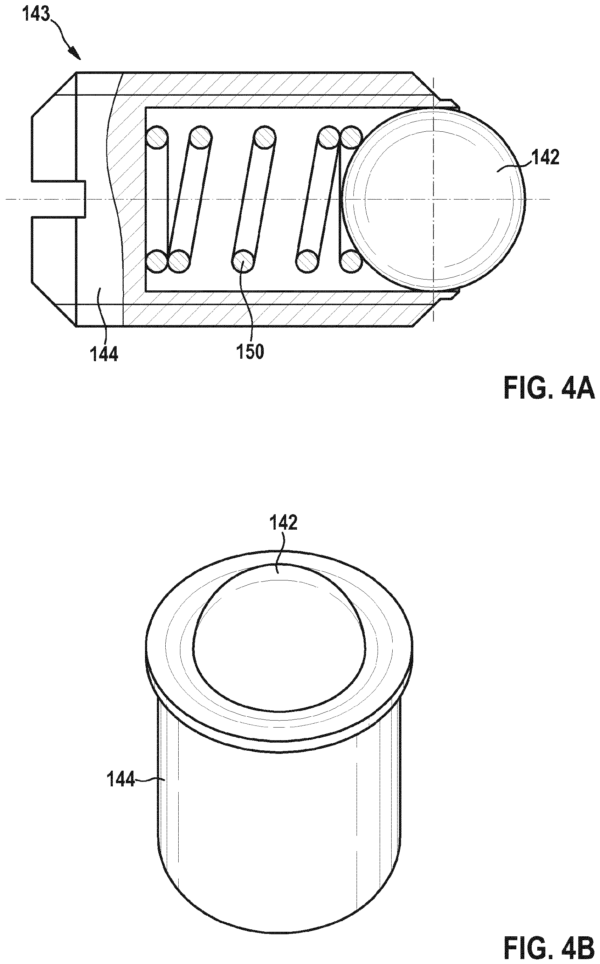

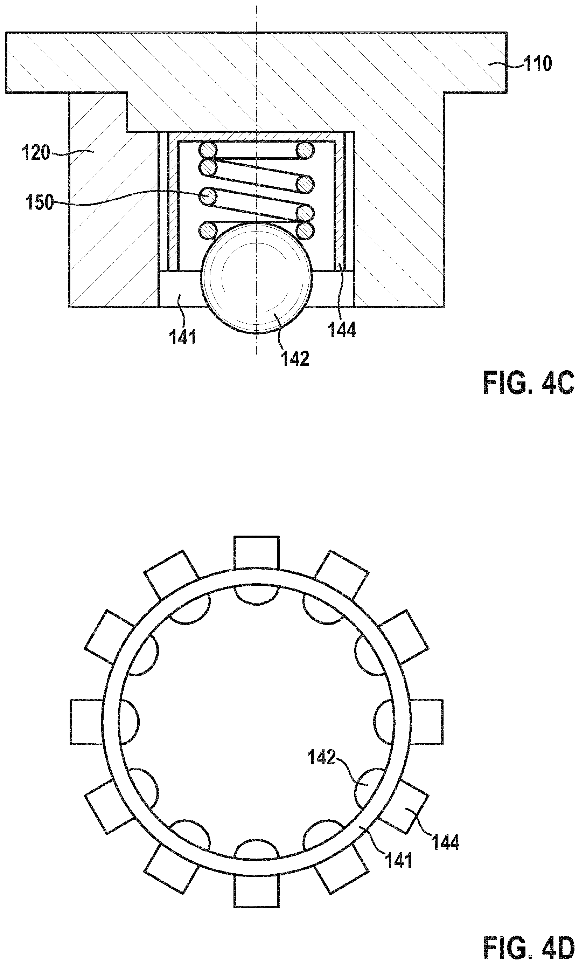

In den Figuren 4a bis 4d sind eine weitere Ausführungsform des erfindungsgemäßen elektrischen Kontaktelements dargestellt. In diesem Fall ist an jedem Kontaktkörper 142, der vorzugsweise als Kugel ausgebildet ist, jeweils ein individuelle Federelement 150 angeordnet, wobei das Federelement 150 innerhalb einer Hülse 144 angeordnet ist, welche zusammen mit der Hülse 144 ein federndes Druckstück 143 bildet (Fig. 4a und 4b). Das Federelement 150 ist dabei vorzugsweise als Schraubenfeder ausgebildet. Wie in Fig. 4c dargestellt ist, ist das federnde Druckstück 143, insbesondere die Hülse 144 und das Federelement in der Nut 111 des äußeren Profilrings 120 bestehend aus Gehäuse 120 und Deckel 130, angeordnet bzw. befestigt. Auch in dieser Ausführungsform wird ein Käfigelement 141 zur Beabstandung und Positionierung der Kontaktkörper 141 und damit auch der Federelemente 150 verwendet (Fig. 4c und 4d).

### Bezugszeichenliste

| | |
|---|---|
| Elektrisches Kontaktbauteil | 100 |
| Außenring/Profilring | 110 |
| Nut Außenring/Profilring | 111 |
| Gehäuse Außenring/Profilring | 120 |
| Steg Gehäuse | 121 |
| Deckel Außenring/Profilring | 130 |
| Steg Deckel | 131 |
| Elektrisches Kontaktelement | 140 |
| Käfigelement | 141 |
| Kontaktkörper (Kugel) | 142 |
| Federndes Druckstück | 143 |
| Druckstückhülse | 144 |
| Federelement | 150 |
| Kontaktbuchse | 200 |
| Stecker/Elektrodenleitung | 300 |

## Patentansprüche

1. Elektrisches Kontaktbauteil (100) für eine Kontaktbuchse (200) eines implantierbaren Medizingeräts, aufweisend:
- ein elektrisches Kontaktelement (140) zur elektrischen Kontaktierung eines elektrischen Gegenkontakts (300), und
- einen elektrisch leitfähigen Außenring (110) mit einer Steckerbohrung zur Aufnahme des elektrischen Gegenkontakts (300), wobei der Außenring (110, 120, 130) auf der Innenseite eine Aufnahmeeinrichtung (111) zur Aufnahme des elektrischen Kontaktelements (140), insbesondere eine Nut, aufweist,
wobei das elektrische Kontaktelement (140) durch eine Vielzahl von Kontaktkörpern (142) und mindestens ein Käfigelement (141) zur Aufnahme der Kontaktkörper (142) gebildet wird,
**dadurch gekennzeichnet,**
**dass** das elektrische Kontaktbauteil (100) weiterhin ein elektrisch leitfähiges Federelement (150) aufweist, das innerhalb der Aufnahmeeinrichtung (111) zur Aufnahme des elektrischen Kontaktelements (140), insbesondere innerhalb der Nut, zwischen den Kontaktkörpern (142) und der Innenseite des Außenrings (110) angeordnet ist, und
das dazu ausgebildet ist, mindestens einen der Kontaktkörper (142) mit dem Außenring (110) und dem elektrischen Gegenkontakt (300) in elektrischen Kontakt zu halten.

2. Elektrisches Kontaktbauteil (100) nach Anspruch 1, **dadurch gekennzeichnet, dass** der Außenring (110) ein Gehäuse (120) und einen Deckel (130) aufweist, wobei das Gehäuse (120) und der Deckel (130) jeweils einen Steg (121, 131) auf der Innenseite aufweisen, und die Stege (121, 131) mit der Innenseite des Gehäuses die Aufnahmeeinrichtung (111) zur Aufnahme des elektrischen Kontaktelements (140), insbesondere die Nut, bilden.

3. Elektrisches Kontaktbauteil (100) nach einem der vorherigen Ansprüche, **dadurch gekennzeichnet, dass** die Aufnahmeeinrichtung (111) zur Aufnahme des elektrischen Kontaktelements (140), insbesondere die Nut, eines der folgenden Formen aufweist: prismaförmig, rechteckig, trapezförmig, dreieckig, viertelrund oder halbrund.

4. Elektrisches Kontaktbauteil (100) nach einem der vorherigen Ansprüche, **dadurch gekennzeichnet, dass** die Kontaktkörper (142) als Kugeln oder Rollen ausgebildet sind, wobei das Käfigelement (141) als Rollenkäfig oder Kugelkäfig ausgebildet ist.

5. Elektrisches Kontaktbauteil (100) nach einem der vorherigen Ansprüche, **dadurch gekennzeichnet, dass** das Federelement (150) als ringförmiges Federelement ausgebildet ist, insbesondere als geschlossenes ringförmiges Federelement, insbesondere als ringförmiges Rohr oder ringförmige Spiralfeder.

6. Elektrisches Kontaktbauteil (100) nach Anspruch 5, **dadurch gekennzeichnet, dass** das ringförmige Federelement (150) eine der folgenden Querschnitte aufweist: rund, halbrund, s-förmig, z-förmig, c-förmig, Ω-förmig oder rechteckig.

7. Elektrisches Kontaktbauteil (100) nach einem der Ansprüche 1 bis 4, **dadurch gekennzeichnet, dass** an jedem Kontaktkörper (142) jeweils ein Federelement (150) angeordnet ist, wobei insbesondere das jeweilige Federelement (150) als federndes Druckstück ausgebildet ist.

8. Elektrisches Kontaktbauteil (100) nach einem der vorherigen Ansprüche, **dadurch gekennzeichnet, dass** das Käfigelement (141) als Blechkäfig oder Massivkäfig ausgebildet ist, wobei insbesondere der Massivkäfig als ringförmiges Band ausgebildet ist, welches eine Vielzahl von Öffnungen zur Aufnahme der Kontaktkörper (142) aufweist, wobei der Durchmesser der Vielzahl von Öffnungen geringer ist, als der Durchmesser der Kontaktkörper (142).

9. Elektrisches Kontaktbauteil (100) nach einem der vorherigen Ansprüche, **dadurch gekennzeichnet, dass** das Käfigelement (141) aus einem der folgenden Stoffe besteht oder umfasst: ein Kunststoff, insbesondere ein Duroplast, ein Thermoplast, ein Elastomer oder eine Kombinationen davon, Edelstahl, insbesondere Chrom-Nickel-Molybdän-Stahl, eine Platiniridiumlegierung, eine Nickel-Kobaltlegierung, insbesondere MP35N, Titan oder Titanlegierung, insbesondere Nitinol.

10. Elektrisches Kontaktbauteil (100) nach einem der vorherigen Ansprüche, **dadurch gekennzeichnet, dass** der Außenring (110) Edelstahl, insbesondere Chrom-Nickel-Molybdän-Stahl, eine Platiniridiumlegierung, eine Nickel-Kobalt-Legierung, insbesondere MP35N, Titan oder eine Titanlegierung umfasst oder daraus besteht.

11. Elektrisches Kontaktbauteil (100) nach einem der vorherigen Ansprüche, **dadurch gekennzeichnet, dass** die Kontaktkörper (142) Edelstahl, insbesondere Chrom-Nickel-Molybdän-Stahl, eine Platiniridiumlegierung, eine Nickel-Kobalt-Legierung, insbesondere MP35N, Titan oder eine Titanlegierung umfasst oder daraus besteht.

12. Elektrisches Kontaktbauteil (100) nach einem der vorherigen Ansprüche, **dadurch gekennzeichnet, dass** das elektrische Kontaktelement (100) 7 bis 15 Kontaktkörper (142) aufweist.

13. Kontaktbuchse (100) für ein implantierbares Medizingerät, aufweisend mindestens ein elektrisches Kontaktbauteil (100) nach einem der Ansprüche 1 bis 12.

14. Implantierbares Medizingerät, aufweisend mindestens ein elektrisches Kontaktbauteil (100) nach einem der Ansprüche 1 bis 12 oder eine Kontaktbuchse (200) nach Anspruch 13.

15. Implantierbares Medizingerät nach Anspruch 14, **dadurch gekennzeichnet, dass** das Medizingerät als Herzschrittmacher, Kardioverter-Defibrillator oder Neurostimulator ausgebildet ist.

## Claims

1. An electrical contact component (100) for a contact socket (200) of an implantable medical device, comprising:
- an electrical contact element (140) for electrically contacting an electrical counter contact (300); and
- an electrically conductive outer ring (110) having a plug borehole for receiving the electrical mating contact (300), the outer ring (110, 120, 130), on the inside, comprising a receiving unit (111) for receiving the electrical contact element (140), in particular a groove,
wherein
the electrical contact element (140) is formed by a plurality of contact bodies (142) and at least one cage element (141) for receiving the contact bodies (142), **characterized in that**
the electrical contact element (140) comprises an electrically conductive spring element (150) being arranged within the receiving unit (111) for receiving the electrical contact element (140), and in particular within the groove, between the contact bodies (142) and the inside of the outer ring (110), and being designed to keep at least one of the contact bodies (142) in electrical contact with the outer ring (110) and the electrical counter contact (300).

2. The electrical contact component (100) according to claim 1, **characterized in that** the outer ring (110) comprises a housing (120) and a lid (130), the housing (120) and the lid (130) each comprising a web (121, 131) on the inside, and the webs (121, 131) forming the receiving unit (111) for receiving the electrical contact element (140), and in particular the groove, together with the inside of the housing.

3. The electrical contact component (100) according to any one of the preceding claims, **characterized in that** the receiving unit (111) for receiving the electrical contact element (140), and in particular the groove, has one of the following shapes: prismatic, rectangular, trapezoidal, semi-circular, quarter-round or triangular.

4. The electrical contact component (100) according to any one of the preceding claims, **characterized in that** the contact bodies (142) are designed as balls or rolls, the cage element (141) being designed as a roll cage or a ball cage.

5. The electrical contact component (100) according to any one of the preceding claims, **characterized in that** the spring element (150) is designed as an annular spring element, in particular as a closed annular spring element, in particular as an annular tube or annular coil spring.

6. The electrical contact component (100) according to claim 5, **characterized in that** the annular spring element (150) has one of the following cross-sections: round, semi-circular, s-shaped, z-shaped, c-shaped, Ω-shaped or rectangular.

7. The electrical contact component (100) according to any one of claims 1 to 4, **characterized in that** a respective spring element (150) is arranged on each contact body (142), the respective spring element (150) being in particular designed as a spring-loaded pressure piece.

8. The electrical contact component (100) according to any one of the preceding claims, **characterized in that** the cage element (141) is designed as a sheet metal cage or a solid cage, the solid cage being in particular designed as an annular strip, which includes a plurality of openings for receiving the contact bodies (142), and the diameter of the plurality of openings being smaller than the diameter of the contact bodies (142).

9. The electrical contact component (100) according to any one of the preceding claims, **characterized in that** the cage element (141) consists of or comprises one of the following materials: a plastic, in particular a thermoset material, a thermoplastic material, an elastomer or a combination thereof, stainless steel, in particular chromium-nickel-molybdenum steel, a platinum-iridium alloy, a nickel-cobalt alloy, in particular MP35N, titanium or a titanium alloy, in particular Nitinol.

10. The electrical contact component (100) according to any one of the preceding claims, **characterized in that** the outer ring (110) comprises or consists of stainless steel, in particular chromium-nickel-molybdenum steel, a platinum-iridium alloy, a nickel-cobalt alloy, in particular MP35N, titanium or a titanium alloy.

11. The electrical contact component (100) according to any one of the preceding claims, **characterized in that** the contact bodies (142) comprise or consist of stainless steel, in particular chromium-nickel-molybdenum steel, a platinum-iridium alloy, a nickel-cobalt alloy, in particular MP35N, titanium or a titanium alloy.

12. The electrical contact component (100) according to any one of the preceding claims, **characterized in that** the electrical contact element (100) comprises 7 to 15 contact bodies (142).

13. A contact socket (100) for an implantable medical device, comprising at least one electrical contact component (100) according to any one of claims 1 to 12.

14. An implantable medical device, comprising at least one electrical contact component (100) according to any one of claims 1 to 12 or a contact socket (200) according to claim 13.

15. The implantable medical device according to claim 14, **characterized in that** the medical device is designed as a cardiac pacemaker, a cardioverter-defibrillator, or a neurostimulator.

## Revendications

1. Composant de contact électrique (100) destiné à un contact femelle (200) d'un appareil médical implantable, présentant :
- un élément de contact électrique (140) permettant le contact électrique d'un contact électrique complémentaire (300), et
- un anneau extérieur (110) conducteur électriquement avec un alésage de connexion pour la réception du contact électrique complémentaire (300), où l'anneau extérieur (110, 120, 130) présente sur la face intérieure un dispositif de réception (111) pour la réception de l'élément de contact électrique (140), notamment une rainure,
dans lequel l'élément de contact électrique (140) est formé par une multiplicité de corps de contact (142) et au moins un élément de cage (141) pour la réception des corps de contact (142),
**caractérisé en ce**
**que** le composant de contact électrique (100) présente en outre un élément ressort (150) conducteur électriquement qui est disposé à l'intérieur du dispositif de réception (111) pour la réception de l'élément de contact électrique (140), en particulier à l'intérieur de la rainure, entre les corps de contact (142) et la face intérieure de l'anneau extérieur (110), et
qui est conçu pour maintenir en contact électrique au moins un des corps de contact (142) avec l'anneau extérieur (110) et le contact électrique complémentaire (300).

2. Composant de contact électrique (100) selon la revendication 1, **caractérisé en ce que** l'anneau extérieur (110) présente un boitier (120) et un couvercle (130), où le boitier (120) et le couvercle (130) présentent respectivement une tige (121, 131) sur la face intérieure, et les tiges (121, 131) forment avec la face intérieure du boitier le dispositif de réception (111) pour la réception de l'élément de contact électrique (140), notamment la rainure.

3. Composant de contact électrique (100) selon l'une des revendications précédentes, **caractérisé en ce que** le dispositif de réception (111) pour la réception de l'élément de contact électrique (140), notamment la rainure, présente l'une des formes suivantes : la forme d'un prisme, la forme d'un rectangle, la forme d'un trapèze, la forme d'un triangle, la forme d'un quart de disque ou la forme d'un demi disque.

4. Composant de contact électrique (100) selon l'une des revendications précédentes, **caractérisé en ce que** les corps de contact (142) sont conçus sous forme de sphères ou de rouleaux, où l'élément de cage (141) est conçu sous forme d'une cage en rouleau ou d'une cage en forme de sphère.

5. Composant de contact électrique (100) selon l'une des revendications précédentes, **caractérisé en ce que** l'élément ressort (150) est conçu sous forme d'élément ressort annulaire, en particulier, sous forme d'élément ressort annulaire fermé, notamment, sous forme de tube de forme annulaire ou d'un ressort spirale de forme annulaire.

6. Composant de contact électrique (100) selon la revendication 5, **caractérisé en ce que** l'élément ressort (150) de forme annulaire présente l'une des sections transversales suivantes : ronde, semi ronde, en forme de s, en forme de z, en forme de c, en forme d'Ω ou rectangulaire.

7. Composant de contact électrique (100) selon l'une des revendications 1 à 4, **caractérisé en ce que** respectivement un élément ressort (150) est disposé sur chaque corps de contact (142), où, en particulier, l'élément ressort (150) respectif est conçu sous forme d'un organe de pression élastique.

8. Composant de contact électrique (100) selon l'une des revendications précédentes, **caractérisé en ce que** l'élément de cage (141) est conçu sous forme d'une cage en tôle ou d'une cage massive, où, en particulier, la cage massive est conçue sous forme d'une bande en forme d'anneau, laquelle présente un grand nombre d'orifices pour la réception des corps de contact (142), où le diamètre du grand nombre d'orifices est inférieur au diamètre des corps de contact (142).

9. Composant de contact électrique (100) selon l'une des revendications précédentes, **caractérisé en ce que** l'élément de cage (141) est constitué ou comprend l'une des substances suivantes : une matière plastique, notamment un duroplastique, un thermoplastique, un élastomère ou une combinaison de ceux-ci, de l'acier inoxydable, notamment de l'acier chrome-nickel-molybdène, un alliage platine-iridium, un alliage nickel-cobalt, notamment du MP35N, du titane ou un alliage de titane, notamment du Nitinol.

10. Composant de contact électrique (100) selon l'une des revendications précédentes, **caractérisé en ce que** l'anneau extérieur (110) comprend de l'acier inoxydable, notamment de l'acier chrome-nickel-molybdène, un alliage platine-iridium, un alliage nickel-cobalt, notamment du MP35N, du titane ou un alliage de titane, ou en est constitué.

11. Composant de contact électrique (100) selon l'une des revendications précédentes, **caractérisé en ce que** les corps de contact (142) comprennent de l'acier inoxydable, notamment de l'acier chrome-nickel-molybdène, un alliage platine-iridium, un alliage nickel-cobalt, notamment du MP35N, du titane ou un alliage de titane, ou en sont constitués.

12. Composant de contact électrique (100) selon l'une des revendications précédentes, **caractérisé en ce que** l'élément de contact (100) électrique présente de 7 à 15 corps de contact (142).

13. Contact femelle (100) pour un appareil médical implantable, présentant au moins un composant de contact électrique (100) selon l'une des revendications 1 à 12.

14. Appareil médical implantable présentant au moins un composant de contact électrique (100) selon l'une des revendications 1 à 12 ou un contact femelle (200) selon la revendication 13.

15. Appareil médical implantable selon la revendication 14, **caractérisé en ce que** l'appareil médical est conçu en tant que stimulateur cardiaque, cardioverteur défibrillateur ou neurostimulateur.
